# EUROPÄISCHE PATENTSCHRIFT

(11) **EP 2 591 086 B1**
(45) Veröffentlichungstag und Bekanntmachung des Hinweises auf die Patenterteilung: **26.12.2018**
(21) Anmeldenummer: 11726088.5
(22) Anmeldetag: 01.06.2011
(51) Int. Cl.: C12M 1/34, C12M 1/38, C12M 3/04, B01L 3/00, G01N 33/58

(54) **VERFAHREN ZUR AKTIVEN HYBRIDISIERUNG IN MICROARRAYS MIT DENATURIERUNGSFUNKTION**
METHOD FOR ACTIVE HYBRIDIZATION IN MICROARRAYS WITH DENATURING FUNCTION
PROCÉDÉ D'HYBRIDATION ACTIVE DANS DES MICRORÉSEAUX AVEC FONCTION DE DÉNATURATION

(30) Priorität: 06.07.2010 DE 102010030962
(43) Veröffentlichungstag der Anmeldung: 15.05.2013
(73) Patentinhaber: Robert Bosch GmbH, 70442 Stuttgart (DE)
(72) Erfinder: STUMBER, Michael, 70825 Korntal-Muenchingen (DE); DAUB, Martina, 71287 Weissach (DE); RUPP, Jochen, 70178 Stuttgart (DE)
(86) Internationale Anmeldenummer: PCT/EP2011/059053
(87) Internationale Veröffentlichungsnummer: WO 2012/004062

(56) Entgegenhaltungen:
- EP-A1- 1 652 912
- DE-A1-102008 002 336
- US-A- 5 508 197
- US-A- 5 587 128
- US-A1- 2004 009 525
- US-A1- 2004 141 856
- US-A1- 2007 254 372
- US-B1- 6 660 517

## Beschreibung

### Stand der Technik

Die vorliegende Erfindung betrifft ein Verfahren zur aktiven Hybridisierung mit einer gesonderten Denaturierungsfunktion.

Microarrays sind als moderne molekularbiologische Untersuchungssysteme bekannt. So erlauben diese die parallele Analyse von mehreren tausend Einzelnachweisen in einer geringen Menge biologischen Probenmaterials. Dementsprechend haben sich verschiedene Formen von Microarrays etabliert, die nach der Art ihrer Interaktionen eingeteilt werden. Hierzu gehören auf der einen Seite nukleinsäurebasierte Microarrays, die z.B. dazu dienen DNA, mRNA oder rRNA bestimmter Gene von Organismen nachzuweisen. Hierzu werden cDNA, Oligonukleotide oder Fragmente von PCR-Produkten die zur mRNA komplementär sind, auf Trägermaterialien gedruckt ("Spotted Microarrays"). Bei den "Oligonukleotid-Microarrays" werden synthetisch hergestellte Oligonukleotide auf z.B. Glasträger aufgebracht. Die als Sonden fungierenden Oligonukleotide erzeugen auf den Trägern eine größtmögliche Informationsdichte auf kleinstem Raumähnlich einem Computerchip, so dass Microarrays auch gerne als "Genchips" oder "Biochips" bezeichnet werden.

Als weitere Ausführungsformen sind Proteinarrays, die z.B. Antigen-Antikörper-, Enzym-Substrat-, Rezeptor-Protein- oder anderen Protein-Protein-Interaktionen nachweisen, bekannt. Auch Bindungen von Nukleinsäuren an Proteine können nachgewiesen und quantifiziert werden.

Beim Einsatz von Microarrays in der Mikrobiologie werden die Biomoleküle aus einer in der Regel aufbereiteten und vorbehandelten Probe auf den Microarray gegeben, wo es an den einzelnen Stellen ("Spots") zu spezifischen Bindungsreaktionen kommt, die dann über das Vorhandensein bestimmter Moleküle, wie z.B. DNA-Stücke, in der Probe Auskunft geben. Voraussetzung für diese Bindungsreaktion ist das Vorliegen der DNA oder RNA als Einzelstränge, so dass ein vorheriger Denaturierungsschritt unumgänglich ist. Bei der allgemein fortschreitenden Automatisierung gibt es eine Vielzahl von Ansätzen, möglichst viele Einzelschritte der Analyse innerhalb einer Geräteeinheit durchzuführen, um so Zeit und Kosten zu sparen. Hierbei kommen unter anderem Micropumpen zum Einsatz, mit denen die extrem kleinen Probenvolumina innerhalb der Geräteeinheiten transportiert werden, siehe hierzu z.B. US 2004/0141856A1.

Der eigentliche Bindungsvorgang bzw. die Hybridisierung an den Sonden des Microarrays kann wiederum mehrere Stunden in Anspruch nehmen, da die Bewegung der Teilchen der Brown'schen Molekularbewegung folgt und damit nur langsam vonstatten geht. Außerdem erreicht dadurch nur ein Teil der in der Probe vorhandenen Zielmoleküle die jeweilige Fängerstruktur, d.h. die Sonden.

Aus dem Dokument US 2004/0009525 A1 ist ein Microarray zur Detektion von Genen unter Verwendung von Nukleinsäureprobensubstraten bekannt.

Aus der Patentschrift US 5,587,128 und der daraus durch Teilung hervorgegangenen Patentschrift US 6,660,517 B1 sind Vorrichtungen zur Amplifikation von vorausgewählten Polyglutiden bekannt.

Aus dem Dokument US 2004/0141856 A1 ist eine Vorrichtung mit einem Mikroreaktor und einer Mikropumpe zur Durchführung von thermischen PCR-Prozessen bekannt.

Aus dem Dokument EP 1 652 912 A1 ist ebenfalls ein Mikroreaktor zur Untersuchung von biologischem Material bekannt.

### Offenbarung der Erfindung

Aufgabe der Erfindung ist es, ein Verfahren bereit zu stellen, dass die Denaturierung der Biomoleküle, z.B. das Aufschmelzen der als Doppelstränge vorliegenden Nukleinsäuren in Einzelstränge, sowie die Bindung der denaturierten Biomoleküle an die Sonden des Microarrays umfasst und die eigentliche Hybridisierung beschleunigt, ohne dass die Empfindlichkeit der Analyse negativ beeinträchtigt wird.

Gegenstand der vorliegenden Erfindung ist somit ein Verfahren zur aktiven Hybridisierung an mindestens einer Sonde eines Microarrays, das die folgenden Schritte umfasst:
a) Bereitstellen einer Probenflüssigkeit mit reaktiven Bestandteilen,
b) Einführen der Probenflüssigkeit in eine Pumpstrecke mit mindestens einer Pumpeinheit, mindestens einer temperaturgesteuerten Denaturierungseinheit und mindestens einem von der Denaturierungseinheit räumlich getrennten temperaturgesteuerten Reaktionsbereich mit mindestens einem Microarray,
c) Pumpen der Probenflüssigkeit,
d) Denaturierung der reaktiven Bestandteile der Probenflüssigkeit in der Denaturierungseinheit und
e) Hybridisierung der denaturierten Bestandteile an mindestens einer Sonde des Microarrays im Reaktionsbereich.

Die Hybridisierung am Microarray findet im Allgemeinen durch Diffusionsbewegung der Biomoleküle in der Probenflüssigkeit statt. Diese Diffusionsbewegung kann durch Schütteln oder Vibration der Probenflüssigkeit unterstützt werden. Hierzu sind verschiedene Systeme wie z.B. der *Maui®Mixer* von *BioMicro Systems Inc*., bekannt. Das erfindungsgemäße Verfahren bietet demgegenüber den entscheidenden Vorteil, dass der Diffusionsprozess durch Pumpbewegungen der Probenflüssigkeit unterstützt wird. Durch das Pumpen kann eine gleichmäßige, kontrollierte Strömung der Probenflüssigkeit erzeugt werden, die dafür sorgt, dass über den Sonden eine stets ausreichende Anzahl an bindenden Teilchen erhalten bleibt. Einer Verarmung an bindenden Teilchen, wie sie bei diffusionsgesteuerten Prozessen stets gegeben ist, wird somit entgegen gewirkt. Der Reaktionsbereich mit dem Microarray kann sowohl direkt von der Probenflüssigkeit angeströmt werden, oder aber innerhalb einer Reaktionskammer angeordnet sein. In jedem Fall muss jedoch eine gleichmäßige, kontrollierte Strömung über der oder den Sonden des Microarrays sicher gestellt werden. Der Reaktionsbereich kann zudem mit mehreren Microarrays bestückt sein, auf denen jeweils eine oder mehrere Sonden angeordnet sind.

Erfindungsgemäß ist die Pumpstrecke als Kreislauf angeordnet, was die gleichmäßige, kontrollierte Strömung der Probenflüssigkeit über den Sonden des Microarrays zusätzlich unterstützt. Die Pumpgeschwindigkeit ist so ausgelegt, dass die Probe mit wenigen mm/min im Reaktionsbereich fließt. Dies unterstützt die ansonsten diffusionslimitierte Hybridisierungsreaktion. Beispielsweise läuft die Hybridisierungsreaktion bei Nukleinsäuren bei ca. 54°C ab.

Weitere positive Effekte ergeben sich dadurch, dass die Denaturierungseinheit vom Reaktionsbereich räumlich getrennt ist, so dass zunächst eine Denaturierung der Einzelstränge vorgenommen werden kann. Im Falle von Nukleinsäuren wird die Probenflüssigkeit zum Aufschmelzen der Doppelstränge in Einzelstränge über einen Bereich höherer Temperatur (z.B. 94°C) geführt. Das Vorliegen der in der Probe vorhandenen Nukleinsäuren als Einzelstränge ist eine Voraussetzung für die darauffolgende Bindung an die Sonden des Microarrays (Hybridisierung) in dem nachfolgend angeordneten Reaktionsbereich. Die Geschwindigkeit sowie die Empfindlichkeit der Bindungsreaktionen an den Spots des Microarrays werden durch die Erhöhung der verfügbaren Einzelstrangmoleküle verbessert. Dies ist insbesondere dann der Fall, wenn die Pumpstrecke kreisförmig aufgebaut ist
und Nukleinsäuren, die bereits wieder oder immer noch als Doppelstrangmoleküle vorliegen, zyklisch einer erneuten Denaturierung und nachfolgenden Hybridisierung zugeführt werden. Somit können die noch ungebundenen Moleküle in einer Folgerunde an die Sonden des Microarrays binden.

In jedem Fall wird durch das vorliegende Verfahren das Bindungsgleichgewicht der Biomoleküle verschoben und die Empfindlichkeit der Analyse gesteigert, so dass auch der Nachweis von wenigen Biomolekülen in einem Probenvolumen möglich wird.

Die räumliche Trennung der Denaturierungseinheit vom Reaktionsbereich macht die Einstellung optimierter Bedingungen für die jeweiligen Reaktionen möglich. Hierbei ist insbesondere die Einstellung einer optimalen Temperatur für die jeweiligen Biomoleküle von entscheidender Bedeutung. Beispielsweise werden für die Denaturierung von Nukleinsäuren Temperaturbereiche von mindestens 90°C, vorzugsweise von 94 bis 98°C, gewählt. Für die Hybridisierungsreaktion von Nukleinsäuren wird die Temperatur im Reaktionsbereich auf z.B. 54°C gehalten. Wichtig ist in jedem Fall die separate Temperaturkontrolle in der Denaturierungseinheit und dem Reaktionsbereich.

In einer weiteren Ausführungsform ist zwischen der Denaturierung der Biomoleküle in der Denaturierungseinheit und der Hybridisierung im Reaktionsbereich ein Abkühlungsschritt vorgesehen, so dass sichergestellt werden kann, dass die denaturierten Biomoleküle mit einer optimalen Temperatur in den Reaktionsbereich eintreten. Die Kühlung kann mit Hilfe einer entsprechend ausgelegten Kühlstrecke erfolgen.

Für die Temperierung der Denaturierungs- und/oder Hybridisierungseinheit sind verschiedene Methoden einsetzbar (z.B. Widerstandsheizer, Infrarot-Heizer, Heißluftgebläse und/oder Peltierelemente). Da die Hybridisierung im Reaktionsbereich im Allgemeinen bei niedrigeren Temperaturen abläuft, muss sichergestellt werden, dass das Probenvolumen aus der Pumpstrecke die gewünschte Zieltemperatur besitzt, wenn es in den Reaktionsbereich mündet. Der Reaktionsbereich verfügt daher über eine zuverlässige Temperaturkontrolle und kann gegebenenfalls genau wie die Denaturierungseinheit temperiert werden.

Da die Hybridisierungsreaktion im Allgemeinen bei niedrigeren Temperaturen als die Denaturierungsreaktion abläuft, steht eine Abkühlung der Probe auf eine bestimmte Hybridisierungstemperatur im Vordergrund. In einem ersten Temperaturbereich werden die Biomoleküle denaturiert und aufgespalten bzw. aufgeschmolzen. Mit einem zweiten, gegenüber dem ersten Temperaturbereich niedrigeren, Temperaturbereich wird die Hybridisierung an den Sonden des Microarrays gewährleistet und mittels einer optimierten Temperaturkontrolle überwacht.

Neben einem Aufbau als Mehrkomponentensystem lässt sich das Verfahren besonders vorteilhaft als Lab-on-a-Chip-System realisieren, die z.B. aus bestimmten Polymeren als Trägersubstrat geformt werden. Dabei kann die Umsetzung der Pumpfunktion durch verschiedene Pumpen erfolgen. Für eine Anwendung in dem vorliegenden Verfahren eignen sich vor allem auf dem Chip integrierte Mikropumpen, z.B. Mikromembranpumpen oder peristaltische Mikropumpen. Als extern betriebene Pumpen sind z.B. Spritzenpumpen oder peristaltische Pumpen einsetzbar. Pumpen dieser Art sind z.B. in der DE 10 2008 002 336 A1 beschrieben und gehören zum Stand der Technik.

In einer weiteren Ausführungsform sind die Denaturierungseinheit und die Pumpeneinheit als eine bauliche Einheit geformt. Dabei erfolgt die Temperatursteuerung über eine beheizbare Pumpe.

Alternativ dazu liegt die Denaturierungseinheit als Mäanderkanal vor, der auf die zur Denaturierung benötigte Temperatur beheizbar ist. An einem solchen Aufbau ist insbesondere die Durchmischung der Probe innerhalb des Kreislaufs vorteilhaft. Durch die damit verbundene verbesserte Homogenität der Probe wird auch die eigentliche Hybridisierungsreaktion verbessert. Der Mäanderkanal weist vorzugsweise eine Mikrostruktur auf, so dass auch diese Komponente in einem Lab-on-a-Chip-System realisiert werden kann.

In einer weiteren Ausführungsform ist der Reaktionsbereich mit einer zweiten Pumpstrecke verbunden, über die dem Reaktionsbereich für die Hybridisierung notwendige Reaktionsflüssigkeiten und/oder Spülflüssigkeiten zugeführt werden können. In einer besonderen Ausführungsform verfügt die zusätzliche Pumpstrecke über Ventile zur Abtrennung der Denaturierungseinheit von der Hybridisierungseinheit. Alternativ können die für die Reaktion notwendigen Substanzen auch über die zentrale Pumpstrecke dem Verfahren zugeführt werden. In jedem Fall ist jedoch mindestens ein entsprechender Zufluss oder Einlass und ein entsprechender Auslass vorgesehen, über die sowohl die Probenflüssigkeit als auch Reaktionsflüssigkeiten und/oder Spülflüssigkeiten zugeführt bzw. abgelassen werden können.

Die Detektion des Microarrays kann sich in einer weiteren Ausführungsform direkt an den Hybridisierungsschritt anschließen, so dass die Hybridisierung und die Detektion des Microarrays in einer Einheit ablaufen können und ggf. baulich zusammengefasst werden. Besonders vorteilhaft ist jedoch ein Lab-on-a-Chip-System.

### Zeichnungen

Weitere Vorteile und vorteilhafte Ausgestaltungen des erfindungsgemäßen Verfahrens werden durch die Abbildungen veranschaulicht und in der nachfolgenden Beschreibung erläutert. Dabei ist zu beachten, dass die Abbildungen nur beschreibenden Charakter haben und nicht dazu gedacht sind, die Erfindung in irgendeiner Form einzuschränken. In den Abbildungen werden folgende Bezugszeichen verwendet:
- 1:: Pumpeinheit
- 2:: Fluide Verbindung
- 3:: Denaturierungseinheit
- 4:: Reaktionsbereich
- 5:: Microarray
- 6:: Einlass
- 7:: Auslass
- 8:: Trägersubstrat

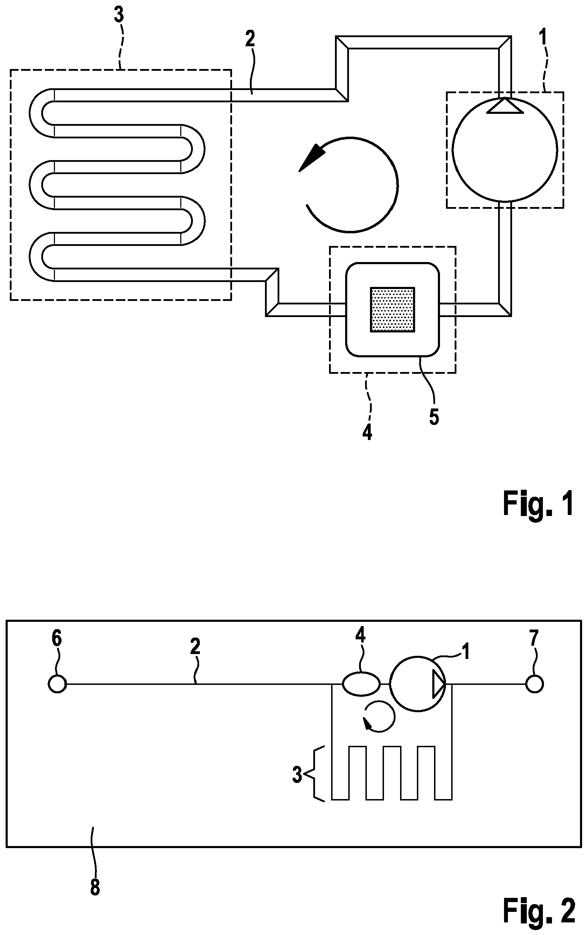

Die Abbildungen 1 und 2 veranschaulichen das erfindungsgemäße Verfahren. Im Einzelnen zeigen die beiden Abbildungen:
- Abb.1 (Fig.1): schematische Darstellung einer Vorrichtung zur Durchführung des Verfahrens, umfassend eine als Kreislauf angeordnete Pumpstrecke mit einer Denaturierungseinheit als Mäanderkanal und einem Reaktionsbereich, der einen Microarray umfasst.
- Abb.1 (Fig.2): schematische Darstellung einer Vorrichtung zur Durchführung des Verfahrens, bei dem der Aufbau als Lab-on-a-Chip-System dargestellt wird, wobei die als Kreislauf angeordnete Pumpstrecke eine Pumpeinheit, eine Denaturierungseinheit und einen Reaktionsbereich umfasst.

Abbildung 1 zeigt einen möglichen Aufbau zur Umsetzung des erfindungsgemäßen Verfahrens. Der Reaktionsbereich **4** samt dem darin enthaltenen Microarray **5** ist über fluide Verbindungen **2** mit einer Pumpstrecke verbunden, in der die Probenflüssigkeit mit Hilfe einer Pumpeinheit **1** im Kreis gepumpt wird- so gekennzeichnet durch den die Fließrichtung angebenden Pfeil in der Mitte der Abbildung. Als Denaturierungseinheit **3** wird ein Mäanderkanal verwendet. Hier wird die Probenflüssigkeit auf einen für die jeweiligen Biomoleküle optimierten Temperaturbereich erhitzt, so dass deren Denaturierung erfolgt. In einem zweiten Temperaturbereich findet anschließend die eigentliche Hybridisierungsreaktion an den Sonden des Microarrays **5** im Reaktionsbereich **4** statt. In der Pumpeinheit **1** kann die Probenflüssigkeit ggf. auf einen dritten Temperaturbereich eingestellt werden.

Abbildung 2 zeigt eine schematische Darstellung einer Vorrichtung zur Durchführung des erfindungsgemäßen Verfahrens als Lab-on-a-Chip-System. Das System umfasst ein Trägersubstrat **8** auf dem ein Einlass **6** und ein Auslass **7** für die Probenflüssigkeit sowie die notwendigen Reagenzien und Spülflüssigkeiten angeordnet sind. Über fluide Verbindungen **2** wird die Probe in die Pumpstrecke mit der Pumpeinheit **1** eingebracht. Die Denaturierungseinheit **3** ist als Mäanderkanal im Micromaßstab ausgeformt. An die Denaturierungseinheit schließt sich der Reaktionsbereich **4** mit dem eigentlichen Microarray **5** zur Hybridisierung der denaturierten Biomoleküle an. Auch in einem Lab-on-a-Chip-System unterliegen die Pumpeinheit **1,** die Denaturierungseinheit **3** und der Reaktionsbereich **4** einer jeweiligen Temperaturkontrolle und/oder Temperaturregelung.

## Patentansprüche

1. Verfahren zur aktiven Hybridisierung an mindestens einer Sonde eines Microarrays (5), umfassend die folgenden Schritte:
a. Bereitstellen einer Probenflüssigkeit mit reaktiven Bestandteilen
b. Einführen der Probenflüssigkeit in eine Pumpstrecke mit mindestens einer Pumpeinheit (1), mindestens einer temperaturgesteuerten Denaturierungseinheit (3) und mindestens einem von der Denaturierungseinheit (3) räumlich getrennten temperaturgesteuerten Reaktionsbereich (4) mit mindestens einem Microarray (5),
c. Pumpen der Probenflüssigkeit,
d. Denaturierung der reaktiven Bestandteile der Probenflüssigkeit in der Denaturierungseinheit (3) und
e. Hybridisierung der denaturierten Bestandteile an mindestens einer Sonde des Microarrays (5) im Reaktionsbereich (4),
**dadurch gekennzeichnet, dass** die Pumpstrecke als Kreislauf angeordnet ist

2. Verfahren nach Anspruch 1, **dadurch gekennzeichnet, dass** die Pumpeinheit (1) und die Denaturierungseinheit (3) eine bauliche Einheit formen.

3. Verfahren nach Anspruch 1 oder 2, **dadurch gekennzeichnet, dass** sich zwischen der Denaturierung in der Denaturierungseinheit (3) und der Hybridisierung im Reaktionsbereich (4) ein Abkühlungsschritt erfolgt.

4. Verfahren nach einem der Ansprüche 1 bis 3, **dadurch gekennzeichnet, dass** sich an die Hybridisierung der denaturierten Bestandteile in Schritt d eine Detektion des Microarrays (5) anschließt.

5. Verfahren nach einem der Ansprüche 1 bis 4, **dadurch gekennzeichnet, dass** die Pumpeinheit (1) eine Mikropumpe, vorzugsweise eine Mikromembranpumpe oder eine peristaltische Mikropumpe, umfasst.

6. Verfahren nach Anspruch 5, **dadurch gekennzeichnet, dass** die Pumpeinheit (1) innerhalb der Pumpstrecke integriert oder off chip angeordnet ist.

7. Verfahren nach einem der Ansprüche 1 bis 6, **dadurch gekennzeichnet, dass** durch das Pumpen in Schritt c. eine gleichmäßige, kontrollierte Strömung der Probenflüssigkeit erzeugt wird.

8. Verfahren nach einem der Ansprüche 1 bis 7, **dadurch gekennzeichnet, dass** die Denaturierungseinheit (3) einen Mäanderkanal (10) umfasst.

9. Verfahren nach einem der Ansprüche 1 bis 8, **dadurch gekennzeichnet, dass** die Denaturierungseinheit (3) mindestens eine Einheit zur Temperierung umfasst.

10. Verfahren nach einem der Ansprüche 1 bis 9, **dadurch gekennzeichnet, dass** der Reaktionsbereich (4) mit einer zweiten Pumpstrecke verbunden ist, über die dem Reaktionsbereich (4) für die Hybridisierung notwendige Reaktionsflüssigkeiten und/oder Spülflüssigkeiten zugeführt werden.

11. Vorrichtung zur Durchführung eines Verfahrens zur aktiven Hybridisierung nach einem der vorhergehenden Ansprüche.

## Claims

1. Method for active hybridization to at least one probe of a microarray (5), comprising the following steps:
a. providing a sample liquid containing reactive constituents,
b. introducing the sample liquid into a pump route comprising at least one pump unit (1), at least one temperature-controlled denaturation unit (3) and at least one temperature-controlled reaction area (4) which is spatially separated from the denaturation unit (3) and has at least one microarray (5),
c. pumping the sample liquid,
d. denaturing the reactive constituents of the sample liquid in the denaturation unit (3) and
e. hybridizing the denatured constituents to at least one probe of the microarray (5) in the reaction area (4),
**characterized in that** the pump route is arranged as a circuit.

2. Method according to Claim 1, **characterized in that** the pump unit (1) and the denaturation unit (3) form a structural unit.

3. Method according to Claim 1 or 2, **characterized in that** a cooling step is effected between the denaturation in the denaturation unit (3) and the hybridization in the reaction area (4).

4. Method according to any of Claims 1 to 3, **characterized in that** detection of the microarray (5) follows the hybridization of the denatured constituents in step d.

5. Method according to any of Claims 1 to 4, **characterized in that** the pump unit (1) comprises a micropump, preferably a micromembrane pump or a peristaltic micropump.

6. Method according to Claim 5, **characterized in that** the pump unit (1) is integrated within the pump route or arranged off chip.

7. Method according to any of Claims 1 to 6, **characterized in that** a uniform, controlled flow of the sample liquid is generated by the pumping in step c.

8. Method according to any of Claims 1 to 7, **characterized in that** the denaturation unit (3) comprises a meandering channel (10).

9. Method according to any of Claims 1 to 8, **characterized in that** the denaturation unit (3) comprises at least one unit for temperature control.

10. Method according to any of Claims 1 to 9, **characterized in that** the reaction area (4) is connected to a second pump route, via which flush liquids and/or reaction liquids required for the hybridization are fed to the reaction area (4).

11. Device for carrying out a method for active hybridization according to any of the preceding claims.

## Revendications

1. Procédé d'hybridation active sur au moins une sonde d'un microréseau (5), comprenant les étapes suivantes :
a. la préparation d'un liquide échantillon contenant des constituants réactifs,
b. l'insertion du liquide échantillon dans une section de pompe comprenant au moins une unité de pompe (1), au moins une unité de dénaturation régulée en température (3) et au moins une zone de réaction (4) régulée en température séparée dans l'espace de l'unité de dénaturation (3), contenant au moins un microréseau (5),
c. le pompage du liquide échantillon,
d. la dénaturation des constituants réactifs du liquide échantillon dans l'unité de dénaturation (3), et
e. l'hybridation des constituants dénaturés sur au moins une sonde du microréseau (5) dans la zone de réaction (4),
**caractérisé en ce que** la section de pompe est agencée sur la forme d'un circuit.

2. Procédé selon la revendication 1, **caractérisé en ce que** l'unité de pompe (1) et l'unité de dénaturation (3) forment une unité structurale.

3. Procédé selon la revendication 1 ou 2, **caractérisé en ce qu'**une étape de refroidissement a lieu entre la dénaturation dans l'unité de dénaturation (3) et l'hybridation dans la zone de réaction (4).

4. Procédé selon l'une quelconque des revendications 1 à 3, **caractérisé en ce que** l'hybridation des constituants dénaturés à l'étape d) est suivie par une détection du microréseau (5).

5. Procédé selon l'une quelconque des revendications 1 à 4, **caractérisé en ce que** l'unité de pompe (1) comprend une micropompe, de préférence une micropompe à membrane ou une micropompe péristaltique.

6. Procédé selon la revendication 5, **caractérisé en ce que** l'unité de pompe (1) est intégrée dans la section de pompe ou agencée hors-puce.

7. Procédé selon l'une quelconque des revendications 1 à 6, **caractérisé en ce qu'**un écoulement uniforme contrôlé du liquide échantillon est généré par le pompage à l'étape c.

8. Procédé selon l'une quelconque des revendications 1 à 7, **caractérisé en ce que** l'unité de dénaturation (3) comprend un canal à méandres (10).

9. Procédé selon l'une quelconque des revendications 1 à 8, **caractérisé en ce que** l'unité de dénaturation (3) comprend au moins une unité pour la régulation de la température.

10. Procédé selon l'une quelconque des revendications 1 à 9, **caractérisé en ce que** la zone de réaction (4) est raccordée avec une deuxième section de pompe, par laquelle des liquides de réaction et/ou des liquides de rinçage nécessaires pour l'hybridation sont introduits dans la zone de réaction (4).

11. Dispositif pour la réalisation d'un procédé d'hybridation active selon l'une quelconque des revendications précédentes.
